Europäisches Patentamt

European Patent Office

Office européen des brevets

(11) Publication number: **0 287 123 B1**

(12) **EUROPEAN PATENT SPECIFICATION**

(45) Date of publication of patent specification: **21.07.93**

(21) Application number: **88106070.1**

(22) Date of filing: **15.04.88**

(51) Int. Cl.⁵: **C12N 1/20**, C12N 15/00, C12P 13/08, //(C12N1/20, C12R1:13),(C12N1/20, C12R1:15)

(54) **L-valine producing microorganism and a process for producing L-valine by fermentation.**

(30) Priority: **16.04.87 JP 93959/87**

(43) Date of publication of application: **19.10.88 Bulletin 88/42**

(45) Publication of the grant of the patent: **21.07.93 Bulletin 93/29**

(84) Designated Contracting States: **DE FR IT**

(56) References cited:
**US-A- 3 893 888**

**CHEMICAL ABSTRACTS, vol. 107, part 1, 6th July 1987, page 539, abstract no. 5741e, Columbus, Ohio, US**

**PATENT ABSTRACTS OF JAPAN, vol. 6, no. 63 (C-99)[941], 22nd April 1982**

(73) Proprietor: **AJINOMOTO CO., INC.**
**5-8, Kyobashi 1-chome, Chuo-ku**
**Tokyo 104(JP)**

(72) Inventor: **Katsurada, Naoki**
**No. 1-1, Suzuki-cho**
**Kawasaki-ku Kawasaki-shi**
**Kanagawa-ken(JP)**
Inventor: **Uchibori, Haruo**
**No. 1-1, Suzuki-cho**
**Kawasaki-ku Kawasaki-shi**
**Kanagawa-ken(JP)**
Inventor: **Tsuchida, Takayasu**
**No. 1-1, Suzuki-cho**
**Kawasaki-ku Kawasaki-shi**
**Kanagawa-ken(JP)**

(74) Representative: **Strehl, Schübel-Hopf, Groening**
**Maximilianstrasse 54 Postfach 22 14 55**
**W-8000 München 22 (DE)**

**Description**

L-valine is an important ingredient for amino acid infusions and for general amino acid formulations. The present invention relates to an improved process for producing L-valine by fermentation.

It is known that microorganisms of the genera Brevibacterium and Corynebacterium may have the ability to produce L-valine when they are provided with the property of requiring L-isoleucine, L-leucine, L-homoserine, etc., or with resistance to norvaline, 2-thiazolealanine, α-aminobutyric acid, etc.

The problem to be solved by the invention is to improve the yield of fermentation and to lower the cost for the production of L-valine.

An object of the present invention is to solve the above problem. In order to find out strains capable of producing L-valine in a higher yield, the inventors have carried out intensive studies for the improvement of known L-valine producing microorganisms of the genera Brevibacterium and Corynebacterium. As a result, among strains exhibiting resistance to polyketides, there were found mutants capable of producing L-valine in a yield higher than those of the prior L-valine producing strains.

Accordingly, there is provided by the present invention a process for producing L-valine which comprises culturing in a liquid medium an L-valine producing microorganism which belongs to the genus Brevibacterium or Corynebacterium and is resistant to polyketides, and then recovering L-valine accumulated in said culture medium.

Many polyketides are already known, including, e.g., triacetic acid lactone, mycophenolic acid, terrin, cerulenin, fallacinal, frenolicin, solorinic acid, and the like.

Microorganisms to be used in the present invention are mutants of the genus Brevibacterium or Corynebacterium having resistance to polyketides and having the ability of producing L-valine. In order to obtain mutants according to the invention, wild strains, such as those set forth hereinbelow, may be provided with the ability of producing L-valine and then rendered resistant to polyketides, or such wild strains may be at first rendered resistant to polyketides and then provided with the ability of producing L-valine.

Wild strains which may be used as a parent strain for the mutants according to the invention include microorganisms of the genera Brevibacterium and Corynebacterium, in particular those known as L-glutamic acid producing strains. Examples of such microorganisms include the following :

Brevibacterium divaricatum ATCC 14,020
Brevibacterium flavum ATCC 14,067
Brevibacterium lactofermentum ATCC 13,869
Brevibacterium saccharolyticum ATCC 14,066
Corynebacterium acetoacidophilum ATCC 13,870
Corynebacterium glutamicum ATCC 13,032

In order to induce the mutation of such a parent strain, there can be utilized any of the ordinary mutation techniques, including the one employing N-methyl-N'-nitro-N-nitrosoguanidine. Mutants according to the invention can be obtained by culturing strains subjected to mutation in a medium containing polyketides and then recovering those capable of growth in said medium.

The mutation induction method employed to obtain the mutants according to the invention is specifically described hereinbelow.

[Method for Inducing Mutation]

Brevibacterium lactofermentum AJ 3434 (FERM-P 1845, resistant to 2-thiazolealanine and requiring isoleucine and methionine) or Corynebacterium glutamicum AJ 3776 (FERM-P 2601) [see Japanese Patent Application (Laid Open) No. 155,684/75] was cultured on a bouillon agar slant at 30 °C for 24 hours, and the cells were suspended in M/30 phosphate buffer to form a suspension containing $10^8$ to $10^9$/ml of cells. To this suspension was added 500 μg/ml of N-methyl-N'-nitro-N-nitrosoguanidine, and the resulting suspension was maintained at 30 °C for 20 minutes. The cells were collected by centrifugation, washed well with M/30 phosphate buffer, inoculated on a medium having the composition set forth below and then cultured at 31.5 °C for a period of from 4 to 10 days.

### COMPOSITION OF THE MEDIUM (PH 7.0)

| Ingredients: | Contents: |
|---|---|
| Glucose | 2.0 g/dl |
| Urea | 0.2 " |
| $(NH_4)_2SO_4$ | 0.5 " |
| $K_2HPO_4$ | 0.1 " |
| $KH_2PO_4$ | 0.1 " |
| $MgSO_4 \cdot 7H_2O$ | 0.001 " |
| $MnSO_4 \cdot 7H_2O$ | 0.001 " |
| Biotin | 50 µg/l |
| Thiamin hydrochloride | 100 " |
| L-isoleucine | 0.3 g/dl |
| DL-methionine | 0.1 " |
| MPA (Mycophenolic acid) | 0.1 " |
| Agar | 2.0 " |

There were obtained Brevibacterium lactofermentum AJ 12,341 (FERM-P 9324) FERM BP-1763 and Corynebacterium glutamicum AJ 12,342 (FERM-P 9325) FERM BP-1764 by selecting colonies capable of growth on the medium and having a high productivity of L-valine. The mutants identified as FERM P-9324 and FERM P-9325 were originally deposited on April 9, 1987 at the Fermentation Research Institute, Agency of Industrial Science and Technology, Ministry of International Trade and Industry (FRI), 1-3, Higashi 1-chome, Tukuba-shi, Ibaragi-ken 305, Japan. The deposits of mutants were then converted into deposits under the Budapest Treaty on February 26, 1988, and were accorded the corresponding FERM BP-1763 and FERM BP-1764.

The resistance to MPA of the thus obtained mutants was compared with that of the parent strains.

Each of the strains was cultured on a slant of a natural medium (containing 1 g/dl of peptone, 1 g/dl of a yeast extract and 0.5 g/dl of NaCl and having a pH of 7.0) for 24 hours, and then the cells were suspended in sterilized water. The suspended cells were inoculated in a medium containing 1.0 g/dl of glucose, 0.15 g/dl of urea, 0.3 g/dl of ammonium sulfate, 0.1 g/dl of $K_2HPO_4$, 0.1 g/dl of $KH_2PO_4$, 0.05 g/dl of $MgSO_4 \cdot 7H_2O$, 0.001 g/dl of $FeSO_4 \cdot 7H_2O$, 0.001 g/dl of $MnSO_4 \cdot 7H_2O$, 50 µg/dl of biotin, 100 µg/l of thiamin hydrochloride, 0.1 g/dl of L-isoleucine, 0.1 g/dl of DL-methionine and MPA in an amount shown in Table 1 and, after 24 hours incubation, the degree of growth of the cells were determined by means of turbidity.

Table 1

| Strain | MPA (%) | | | | |
|--------|---------|---------|------|------|-----|
| | 0 | 0.005 | 0.01 | 0.05 | 0.1 |
| AJ 3434 | 100 | 90 | 35 | 0 | 0 |
| AJ 12341 (FERM BP-1763) | 100 | 100 | 82 | 30 | 5 |
| AJ 3776 | 100 | 85 | 20 | 0 | 0 |
| AJ 12342 (FERM BP-1764) | 100 | 100 | 65 | 5 | 0 |

In many cases, a further improved yield can be attained by additionally providing the above mutants with such properties as resistance to norvaline, resistance to 2-thiazolealanine, resistance to $\alpha$-amonobutyric acid, etc., which are already known to be effective for the improvement of productivity of L-valine.

The microorganisms can be cultured in an ordinary medium containing carbon sources, nitrogen sources, inorganic ions and, where necessary, trace amounts of other organic nutrients.

As carbon sources, there can be used glucose, sucrose, organic acids (such as acetic acid), and the like.

As to nitrogen sources, the use of ammonia gas, ammonium salts, urea, etc. can be preferable.

Their cultivation is preferably carried out under aerobic conditions. Better results can be attained if, during the cultivation, the pH of the medium is maintained at 4 to 8 and its temperature at 25 to 37 °C.

Thus, a large amount of L-valine can be accumulated in the medium after 1 to 7 days of cultivation.

The thus formed L-valine can be recovered from the medium by a conventional method utilizing, e.g., an ion exchange resin.

Example 1

The pH of a seed medium containing 3 g/dl of glucose, 0.1 g/dl of $KH_2PO_4$, 0.04 g/dl of $MgSO_4 \cdot 7H_2O$, 0.001 g/dl of $MnSO_4 \cdot 7H_2O$, 0.3 g/dl of urea, 0.13 g/dl (as total nitrogen) of hydrolysate of bean protein, 10 $\mu$g/dl of biotin, 300 $\mu$g/l of thiamin HCl and 0.01 g/dl of DL-methionine was adjusted to 6.5. Into shouldered 500 ml flasks were placed 50 ml each of the seed medium, and then the medium was sterilized. Each of the flasks was inoculated with a strain shown in Table 1, and its culturing was carried out with shaking for 20 hours, during which the temperature of the medium was maintained at 31.5 °C.

Into 1 liter fermenters was placed 285 ml each of a medium (adjusted to a pH of 6.5) containing 14 g/dl of glucose, 0.1 g/dl of $KH_2PO_4$, 0.04 g/dl of $MgSO_4 \cdot 7H_2O$, 0.001 g/dl of $FeSO_4 \cdot 7H_2O$, 0.001 g/dl of $MnSO \cdot 7H_2O$, 2.0 g/dl of ammonium sulfate, 0.08 g/dl (as total nitrogen) of hydrolysate of soybeans, 5 $\mu$g/dl of biotin, 30 $\mu$g/l of thiamin HCl and 60 mg/dl of DL-methionine, and the contents were sterilized. To the resulting medium was inoculated with 15 ml each of the above seed culture. Its culturing was carried out with agitation and aeration at a temperature of 31.5 °C until the concentration of glucose decreased to 0.5 g/dl or less, during which the pH of the medium was maintained at 6.5 to 7.0 by the addition of ammonia gas.

There were obtained L-valine at yields (based on the weight of glucose) shown in Table 2.

| Table 2 | |
|---|---|
| Tested Strain | Yield of L-Valine (%) |
| AJ 3434 | 20 |
| AJ 12341 | 28 |
| AJ 3776 | 19 |
| AJ 12342 | 26 |

Brevibacterium lactofermentum AJ 12341 was cultured in the same manner as above. There was obtained 1 liter of cultured broth which was then subjected to centrifugation to remove the cells. The supernatant obtained was passed through a column of a strongly acidic ion exchange resin (Dia Ion SK-IB, $NH_4^+$ type). The resin was washed with water and eluted with 2N ammonia water, and the eluent was condensed to obtain 19 g of L-valine.

Example 2

Into 1 liter jar fermenters was charged 300 ml each of a medium for main fermentation having the composition set forth below, and then the contents of the fermenters were sterilized.

| Compositions of Fermentation Media: | | |
|---|---|---|
| | Seed Medium | Medium for Main Fermentation |
| Glucose (g/dl) | 1.5 | -- |
| Ammonium acetate (g/dl) | 0.3 | 0.3 |
| $KH_2PO_4$ (g/dl) | 0.15 | 0.2 |
| $MgSO_4 \cdot 7H_2O$ (g/dl) | 0.04 | 0.1 |
| $Fe^{++}$ (ppm) | 2 | 2 |
| $Mn^{++}$ (ppm) | 2 | 2 |
| Hydrolysate of soybeans (g/ l) (Total nitrogen, 6.4 g/dl) | 3 | 2 |
| Biotin ($\gamma$/l) | 100 | 100 |
| Vitamin $B_1$ Hydrochloride ($\gamma$/l) | 100 | 100 |
| Ammonium sulfate (g/dl) | -- | 1 |
| Urea (g/dl) | 0.2 | -- |
| DL-methionine (mg/dl) | 60 | 60 |
| pH | 8.0 | 7.2 |

The strains set forth in Table 3 were cultivated with shaking for 24 hours in the above-described seed medium, and the seed cultures obtained were separately inoculated in each medium contained in the fermenters at a percentage of 5% and cultured at 31 °C with a stirring of 1,500 rpm and an aeration of 1/1 VVM.

During the cultivation, a 1:0.20 (by weight) of acetic acid and ammonium acetate (concentration of acetic acid, 70 g/dl) was added to the medium so as to maintain its pH at 7.5 and, at the same time, to supply the carbon source. After 90 hours of cultivation, L-valine was accumulated at yields shown in Table 3 (based on the weight of acetic acid consumed). After the completion of the fermentation by strain AJ 12341, the cells were removed by the conventional method to obtain 15 g of crude crystals of L-valine, per liter of culture medium.

Table 3

| Tested Strain | Yield (%) of L-Valine Based on the Weight of Acetic Acid |
|---------------|-----------------------------------------------|
| AJ 3434 | 8 |
| AJ 12341 | 12 |
| AJ 3776 | 7 |
| AJ 12342 | 10 |

**Claims**

1. Microorganism of the genus Brevibacterium or Corynebacterium having high productivity of L-valine, characterised by being resistant to polyketides.

2. Microorganism according to claim 1, further characterised by its ability of producing L-glutamic acid.

3. Microorganisms Brevibacterium lactofermentum FERM-BP 1763 and Corynebacterium glutamicum FERM-BP 1764

4. A process for producing a microorganism according to any of claims 1 to 3, characterised in that a wild strain of the genus Brevibacterium or Corynebacterium is subjected to a conventional mutation treatment to be first provided with the ability of producing L-valine and then rendered resistant to polyketides or at first rendered resistant to polyketides and then provided with the ability of producing L-valine.

5. A process for producing a microorganism according to any of claims 1 to 3, characterised in that an L-valine producing microorganism of the genus Brevibacterium or Corynebacterium is subjected to a conventional mutation step to be rendered resistant to polyketides.

6. A process according to claim 4 or 5, characterised in that the intended mutants are screened by cultivating the strains in a medium containing polyketides and recovering those capable of growing in said medium.

7. A process for producing L-valine by fermentation which comprises culturing in a liquid culture medium an L-valine producing microorganism of the genus Brevibacterium or Corynebacterium and recovering L-valine accumulated in said culture medium, characterised in that the L-valine producing microorganism is a mutant strain according to any of claims 1 to 3.

**Patentansprüche**

1. Mikroorganismus des Genus Brevibacterium oder Corynebacterium, mit einer hohen Produktivität für L-Valin, dadurch gekennzeichnet, daß er resistent gegen Polyketide ist.

2. Mikroorganismus gemäß Anspruch 1, außerdem gekennzeichnet durch seine Fähigkeit L-Glutaminsäure zu produzieren.

6

**3.** Mikroorganismen Brevibacterium lactofermentum FERM-BP 1763 und Corynebacterium glutamicum FERM-BP 1764.

**4.** Verfahren zur Herstellung eines Mikroorganismus gemäß einem der Ansprüche 1 bis 3, dadurch gekennzeichnet, daß ein Wildtyp des Genus Brevibacterium oder Corynebacterium einer üblichen Mutations-Behandlung unterzogen wird, um zunächst mit der Fähigkeit L-Valin zu produzieren, versehen zu werden und dann resistent gegen Polyketide gemacht wird oder zunächst resistent gegen Polyketide gemacht zu werden, und dann mit der Fähigkeit L-Valin zu produzieren, versehen wird.

**5.** Verfahren zur Herstellung eines Mikroorganismus gemäß einem der Ansprüche 1 bis 3, dadurch gekennzeichnet, daß ein L-Valin produzierender Mikroorganismus des Genus Brevibacterium oder Corynebacterium einem üblichen Mutationsschritt unterzogen wird, um resistent gegen Polyketide gemacht zu werden.

**6.** Verfahren gemäß Anspruch 4 oder 5, dadurch gekennzeichnet, daß die gewünschten Mutanten durch Kultivierung der Stämme in einem Polyketide enthaltenden Medium und Gewinnen der in diesem Medium zum Wachstum befähigten selektiert werden.

**7.** Verfahren zur Herstellung von L-Valin durch Fermentation, welches die Züchtung eines L-Valin produzierenden Mikroorganismus des Genus Brevibacterium oder Corynebacterium in einem flüssigen Kulturmedium und die Gewinnung des im Kulturmedium angereicherten L-Valins umfaßt, dadurch gekennzeichnet, daß der L-Valin produzierende Mikroorganismus ein Mutantenstamm gemäß einem der Ansprüche 1 bis 3 ist.

**Revendications**

**1.** Microorganisme du genre Brevibacterium ou Corynebacterium ayant une productivité élevée de L-valine, caractérisé en ce qu'il est résistant aux polycétides.

**2.** Microorganisme selon la revendication 1, caractérisé en outre par sa capacité à produire de l'acide L-glutamique.

**3.** Microorganismes Brevibacterium lactofermentum FERM-BP 1763 et Corynebacterium glutamicum FERM-BP 1764.

**4.** Procédé de production d'un microorganisme selon l'une quelconque des revendications 1 à 3, caractérisé en ce qu'on soumet une souche sauvage du genre Brevibacterium ou Corynebacterium à un traitement de mutation classique pour lui donner d'abord la capacité de produire de la L-valine puis la rendre résistante aux polycétides, ou pour la rendre d'abord résistante aux polycétides puis lui donner la capacité de produire de la L-valine.

**5.** Procédé de production d'un microorganisme selon l'une quelconque des revendications 1 à 3, caractérisé en ce qu'on soumet un microorganisme producteur de L-valine du genre Brevibacterium ou Corynebacterium à une étape de mutation classique pour le rendre résistant aux polycétides.

**6.** Procédé selon la revendication 4 ou 5, caractérisé en ce qu'on crible les mutants recherchés en cultivant les souches dans un milieu contenant des polycétides et en recueillant celles capables de croître sur un tel milieu.

**7.** Procédé de production de L-valine par fermentation, qui comprend la culture dans un milieu de culture liquide d'un microorganisme producteur de L-valine du genre Brevibacterium ou Corynebacterium et la récupération de la L-valine accumulée dans ledit milieu de culture, caractérisé en ce que le microorganisme producteur de L-valine est une souche mutante selon l'une quelconque des revendications 1 à 3.